# DEMANDE DE BREVET EUROPEEN

(11) **EP 4 382 899 A1**
(43) Date de publication de la demande: **12.06.2024**
(21) Numéro de dépôt: 23214287.7
(22) Date de dépôt: 05.12.2023
(51) Int. Cl.: G01N 27/20, G01N 33/20, G01R 27/00, H01M 4/00, G01N 27/04, G01R 1/00

(54) **MÉTHODES ET SYSTÈMES DE MESURE DE LA CONDUCTIVITÉ ÉLECTRONIQUE D'ÉLECTRODES POUR BATTERIE**

(30) Priorité: 05.12.2022 FR 2212789
(71) Demandeur: Verkor, 38000 Grenoble (FR)
(72) Inventeur: FARKHONDEH BORAZJANI, Mohammad, 38000 Grenoble (FR); NGUYEN, Tuan Tu, 38000 Grenoble (FR); DELOBEL, Bruno, 38000 Grenoble (FR)
(74) Mandataire: Nasr, Abdelmottaleb

(57) **Abrégé**

Système (1) de mesure de conductivité électronique d'une bande (2) d'électrode défilant entre un premier et un deuxième rouleau (3, 4) rotatif, ce système comprenant
- un dispositif (11) de mesure de conductivité électronique ;
- un premier élément conducteur (12) de contact mobile en rotation connecté audit dispositif, ce premier élément conducteur de contact intégrant un premier bord distal circulaire destiné à venir en contact tangentiel avec une première surface de la bande d'électrode ;
- un deuxième élément conducteur (13) de contact mobile en rotation connecté audit dispositif de mesure, ce deuxième élément conducteur de contact intégrant un deuxième bord distal circulaire destiné à venir en contact tangentiel avec la première surface lorsque le premier bord distal circulaire est en contact avec ladite première surface de sorte à pouvoir appliquer un courant électrique prédéfini entre le premier et le deuxième élément conducteur (12, 13).de contact

## Description

La présente invention a trait aux méthodes et systèmes de contrôle des performances électrochimiques des électrodes pour batterie, et plus particulièrement aux méthodes et systèmes de mesure de la conductivité électronique de telles électrodes.

La conductivité électronique d'une électrode représente son aptitude à permettre le libre déplacement des charges électriques entre le matériau actif et le collecteur du courant de cette électrode. Plus cette conductivité électrique est élevée, plus le transfert des charges électriques lors des réactions électrochimiques de l'électrode est important. Diverses stratégies visant à améliorer cette propriété fondamentale de l'électrode existent, telles que le choix des matériaux de l'électrode ou l'ajout d'un agent ou additif conducteur au matériau actif.

Une fois une composition d'électrodes conférant une certaine conductivité électronique est adoptée, cette dernière est régulièrement vérifiée sur des électrodes échantillons prises en bout de la chaîne de fabrication ou sur des pastilles découpées à partir de celles-ci. Pour cela, un courant électrique est appliqué entre deux points sur la surface de l'électrode afin de mesurer l'évolution de la tension électrique entre ces deux points en fonction du temps. L'évolution de la tension en fonction du courant électrique appliqué représente la conductivité électronique de l'électrode. Il est également connu de mesurer cette conductivité électronique par la méthode des « quatre pointes ».

Cependant, un inconvénient majeur de ce contrôle de la conductivité électronique est que des électrodes échantillons ne permettent pas de donner un renseignement précis et complet sur l'état de la conductivité électronique de chacune des électrodes produites. Des électrodes échantillons, aussi fréquentes soient-elles, ne sont nécessairement pas représentatives de la totalité des électrodes produites. La conductivité électronique est, en effet, sujet à de nombreux facteurs tels qu'une qualité variable des matériaux utilisés, une variation dans la teneur de l'additif conducteur, la qualité de dépôt du matériau actif sur le collecteur de courant ou, plus généralement, le moindre défaut accidentel ou répétitif dans l'une quelconque des étapes du processus de fabrication d'électrode pour batterie (préparation du matériau actif, préparation du collecteur de courant, dépôt du matériau actif sur le collecteur de courant, séchage de l'électrode, calandrage et découpage de l'électrode). Un écart accidentel ou aléatoire de la conductivité électronique objective peut ainsi se produire sans qu'il ne soit détecté ou détecté trop tard. En outre, entre le moment où l'on prélève une électrode échantillon et celui où l'on constate ex situ que cet échantillon ne répond pas aux critères de qualité imposés en termes de conductivité électronique (un défaut ponctuel ou de masse), la fabrication du produit se poursuit alors que la production effectuée pendant ce délai est inutilisable. Autrement dit, un contrôle d'électrodes échantillons ne permet pas un contrôle correctif immédiat, ce qui entraîne une perte des électrodes en cours de production et, par conséquent, d'éventuels dommages financiers et environnementaux.

Un autre inconvénient est que le contrôle de la conductivité électronique porte sur des électrodes finies de sorte qu'un défaut de conductivité électronique n'est confirmé qu'au stade de l'électrode finie, notamment découpées et adaptées au format de la batterie pour laquelle elles sont destinées. Cette approche ne permet pas de prévenir les défauts de masse et réduire le surcoût de fabrication des électrodes.

Un objet de la présente invention est de remédier aux inconvénients précités.

Un autre objet de la présente invention est de proposer des méthodes et systèmes de mesure de la conductivité électronique des électrodes pour batterie capables d'empêcher des défauts et, par conséquent, de réduire le coût de fabrication des électrodes pour batterie.

A cet effet, il est proposé, en premier lieu, un ensemble de systèmes de mesure de conductivité électronique d'une bande d'électrode pour batterie défilant entre un premier et un deuxième rouleau rotatif et comprenant une couche de matériau actif sur ses deux faces, cet ensemble intégrant un premier système de mesure de conductivité électronique comprenant
- un dispositif de mesure de conductivité électronique ;
- un premier élément conducteur de contact connecté audit dispositif de mesure, ce premier élément conducteur de contact intégrant un premier bord distal circulaire destiné à venir en contact sensiblement tangentiel avec une première surface d'une première face desdites deux faces de la bande d'électrode ;
- un deuxième élément conducteur de contact connecté audit dispositif de mesure, ce deuxième élément conducteur de contact intégrant un deuxième bord distal circulaire destiné à venir en contact sensiblement tangentiel avec la première surface lorsque le premier bord distal circulaire est en contact avec ladite première surface de sorte à pouvoir appliquer un courant électrique prédéfini entre le premier élément conducteur de contact et le deuxième élément conducteur de contact ;
- un support pour le premier élément conducteur de contact et le deuxième élément conducteur de contact;
- le premier élément conducteur de contact étant monté mobile en rotation sur ledit support de sorte que, lorsque le premier bord distal circulaire vient en contact sensiblement tangentiel avec ladite première surface de la bande d'électrode défilant entre le premier rouleau rotatif et le deuxième rouleau rotatif, ledit premier élément conducteur de contact soit entrainé en rotation;
- le deuxième élément conducteur de contact étant monté mobile en rotation sur ledit support de sorte que, lorsque le deuxième bord distal circulaire vient en contact sensiblement tangentiel avec ladite première surface de la bande d'électrode défilant entre le premier rouleau rotatif et le
deuxième rouleau rotatif, le deuxième élément conducteur de contact soit entrainé en rotation ; ledit ensemble intégrant, en outre, un deuxième système de mesure de conductivité électronique identique audit premier système de mesure de conductivité électronique, le premier système et le deuxième système étant disposés en regard l'un de l'autre de part et d'autre de la bande d'électrode.

Diverses caractéristiques supplémentaires peuvent être prévues, seules ou en combinaison :
- le premier bord distal circulaire et/ou le deuxième bord distal circulaire du premier système sont de forme arrondie;
- le premier élément conducteur de contact et/ou le deuxième élément conducteur de contact du premier système sont de forme sphérique montés en liaison rotule sur ledit support ;
- la distance entre le premier bord distal circulaire et le deuxième bord distal circulaire du premier système est réglable ;
- le premier système comprend, en outre,
   un troisième élément conducteur de contact intégrant un troisième bord distal circulaire destiné à venir en contact sensiblement tangentiel avec ladite première surface lorsque le premier et le deuxième bord distal circulaire sont en contact avec ladite première surface ;
   un quatrième élément conducteur de contact intégrant un quatrième bord distal circulaire destiné à venir en contact sensiblement tangentiel avec la première surface lorsque le premier, le deuxième et le troisième bord distal circulaire sont en contact avec ladite première surface de sorte à pouvoir mesurer une tension électrique entre le troisième élément conducteur de contact et le quatrième élément conducteur de contact, le troisième et le quatrième bord distal circulaire étant agencés de sorte à venir en contact avec ladite première surface entre le premier et le deuxième bord distal circulaire sensiblement sur une même droite;
   le troisième élément conducteur de contact étant monté mobile en rotation sur ledit support de sorte que, lorsque le troisième bord distal circulaire vient en contact sensiblement tangentiel avec ladite première surface de la bande d'électrode défilant entre le premier rouleau rotatif et le deuxième rouleau rotatif, le troisième élément conducteur de contact soit entrainé en rotation;
   le quatrième élément conducteur de contact étant monté mobile en rotation sur ledit support de sorte que, lorsque le quatrième bord distal circulaire vient en contact sensiblement tangentiel avec ladite première surface de la bande d'électrode défilant entre le premier rouleau rotatif et le deuxième rouleau rotatif, le quatrième élément conducteur de contact soit entrainé en rotation ;
   - le dispositif de mesure de conductivité électronique du premier système est configuré pour
   mesurer une conductivité électronique entre le premier élément conducteur de contact et le deuxième élément conducteur de contact ;
   générer un message d'alerte lorsque la conductivité électronique mesurée est en dehors d'un intervalle prédéfini ;
- le premier système comprend, en outre, un moyen apte à situer un défaut de conductivité électronique.

Il est proposé, en deuxième lieu, une méthode de mesure de conductivité électronique d'une bande d'électrode pour batterie comprenant une couche de matériau actif sur ses deux faces, cette méthode comprenant :
- une étape de défilement de ladite bande d'électrode entre un premier rouleau rotatif et un deuxième rouleau rotatif;
- une étape de mesure d'une conductivité électronique de la bande d'électrode au moyen de l'ensemble de systèmes de mesure présenté ci-dessus disposé entre le premier rouleau rotatif et le deuxième rouleau rotatif.

Dans un mode de réalisation, cette méthode comprend, en outre, une étape de découpe de ladite bande d'électrode en une pluralité d'électrodes en aval de l'étape de mesure par rapport au sens de défilement de cette bande d'électrode.

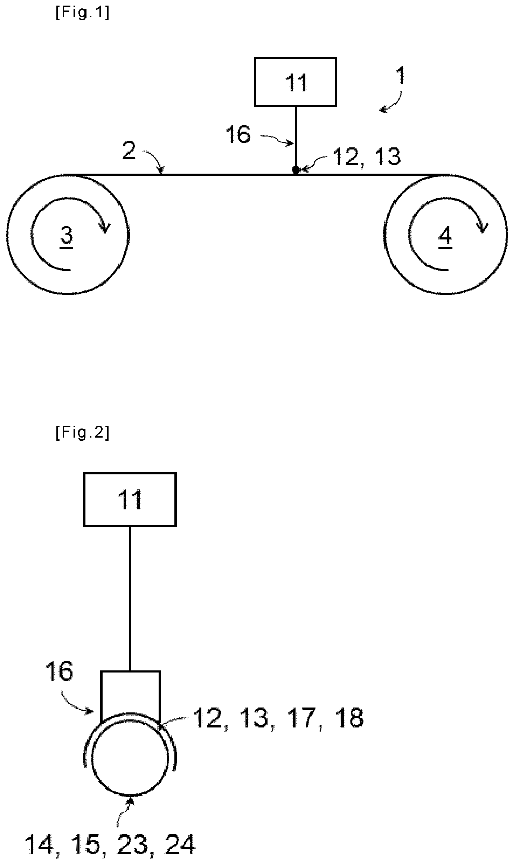

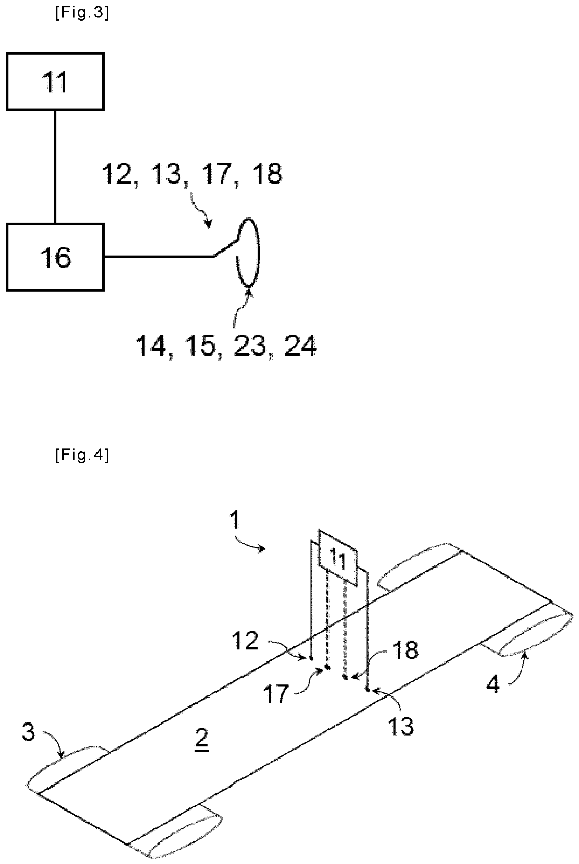

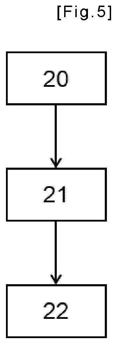

D'autres caractéristiques et avantages de l'invention apparaîtront plus clairement et de manière concrète à la lecture de la description ci-après de modes de réalisation, laquelle est faite en référence aux dessins annexés dans lesquels :
la figure [Fig.1] illustre schématiquement un système de mesure de conductivité électronique d'une bande d'électrode selon divers modes de réalisation ;
la figure [Fig.2] illustre schématiquement des éléments du système de mesure selon un premier mode de réalisation ;
la figure [Fig.3] illustre schématiquement des éléments du système de mesure selon un deuxième mode de réalisation ;
la figure [Fig.4] illustre schématiquement un système de mesure quatre pointes de conductivité électronique d'une bande d'électrode selon divers modes de réalisation ;
la figure [Fig.5] illustre schématiquement des étapes d'une méthode de mesure de conductivité électronique d'une bande d'électrode selon divers modes de réalisation.

En se référant à la figure 1, il est affiché un système **1** de mesure de conductivité électronique d'une bande **2** d'électrode pour batterie (ou, plus généralement, pour un dispositif de stockage électrochimique de l'énergie) défilant entre un premier rouleau **3** rotatif et un deuxième rouleau **4** rotatif.

La bande **2** d'électrode est une bande continue d'un métal revêtu d'une couche de matériau actif sur au moins une de ses deux faces opposées. Dans un mode de réalisation, la bande **2** d'électrode est à double revêtement. En d'autres termes, chaque face de la bande de métal est revêtue de matériau actif.

Le premier et le deuxième rouleau **3, 4** rotatif sont deux rouleaux de déroulement, d'avancement ou de convoyage de la bande **2** d'électrode dont les axes de rotation sont sensiblement parallèles. Lors de son défilement entre le premier et le deuxième rouleau **3, 4** rotatif, la bande **2** d'électrode est maintenue tendue. Les rouleaux **3, 4** rotatifs maintiennent, en effet, une certaine tension sur la bande **2** d'électrode de sorte à garantir une planéité de la bande **2** d'électrode en défilement. Les premier et deuxième rouleaux **3,4** se situent en amont d'un poste de découpe des électrodes. La mesure de la conductivité électronique de la bande **2** d'électrode peut, ainsi, être réalisée en ligne sans interrompre le procédé de fabrication d'électrodes.

Le système **1** de mesure comprend un dispositif **11** (ou un instrument) de mesure de conductivité électronique auquel sont connectés au moins un premier élément conducteur **12** de contact et un deuxième élément conducteur **13** de contact destinés à venir en contact avec une surface de la bande **2** d'électrode. Une pluralité de paires d'éléments conducteurs **12, 13** de contact connectés au dispositif **11** de mesure peut être envisagée. Avantageusement, une pluralité de paires d'éléments conducteurs **12, 13** de contact permet d'obtenir une discrétisation de la mesure. En effet, un défaut est, le plus souvent, local, d'où l'intérêt de mesurer, de préférence, sur toute la largeur de la bande **2** d'électrode. Des éléments conducteurs **12, 13** de contact sont connectés par paires au dispositif **11** de mesure de conductivité électronique. La conductivité électronique de la bande **2** d'électrode peut être mesurée directement par le dispositif **11** de mesure ou déduite d'une autre grandeur physique mesurée telle que la résistance et/ou l'impédance.

Chacun des éléments conducteurs **12, 13** de contact comprend respectivement un bord distal **14, 15** (un bout ou une extrémité de contact) circulaire destiné à venir en contact sensiblement tangentiel avec une surface de la bande **2** d'électrode. Chacun des éléments conducteurs **12, 13** de contact se termine par un bord sphérique ou circulaire pour assurer un contact sensiblement tangentiel avec une surface de la bande **2** d'électrode en défilement. Il en résulte, avantageusement, un contact sensiblement ponctuel avec la bande **2** d'électrode en défilement. La géométrie circulaire des bords distaux **14, 15** destinés à venir en contact avec la bande **2** d'électrode permet, avantageusement, d'éviter la détérioration du matériau actif.

Afin d'appliquer un courant électrique prédéfini entre le premier élément conducteur **12** et le deuxième élément conducteur **13** de contact, le premier bord distal **14** et le deuxième bord distal **15** sont, simultanément, mis en contact avec la bande **2** d'électrode en défilement. Lorsqu'ils sont en contact avec la surface de la bande **2** d'électrode, les éléments conducteurs **12, 13** de contact ferment un circuit de mesure de conductivité électronique. Un contact électrique s'établit entre le premier et le deuxième élément conducteur **12, 13** de contact via la surface de la bande **2** d'électrode. En mesurant ce courant, le dispositif **11** de mesure détermine la conductivité électronique de la section cible de la surface de la bande **2** d'électrode.

Chacun des éléments conducteurs **12, 13** de contact est monté mobile en rotation sur un support **16** du système **1** de mesure de conductivité électronique de sorte que cet élément conducteur **12, 13** de contact soit entrainé en rotation lorsque son bord distal **14, 15** circulaire vient en contact sensiblement tangentiel avec une surface de la bande **2** d'électrode défilant entre le premier et le deuxième rouleau **3, 4** rotatif. En étant libre en rotation, l'élément conducteur **12, 13** de contact suit le mouvement de la bande **2** d'électrode. Il en résulte, avantageusement, que chacun des éléments conducteurs **12, 13** de contact est en contact roulant avec la surface de la bande **2** d'électrode de sorte à ne pas détériorer la couche de matériau actif. Un contact roulant avec la surface de la bande **2** d'électrode permet, avantageusement, de réaliser une mesure continue et en ligne de sa conductivité électronique pendant son défilement entre les deux rouleaux **3, 4** rotatifs. Une mesure in situ de la conductivité électronique de la bande **2** d'électrode sur toute sa longueur avant son découpage est ainsi possible.

Dans un premier mode de réalisation illustré sur la figure 2, les éléments conducteurs **12, 13** de contact sont de forme sphérique et montés mobiles en rotation sur le support **16.** Ainsi, les éléments conducteurs **12, 13** de contact sont montés en liaison rotule sur le support **16.** Dans un deuxième mode de réalisation illustré par la figure 3, les éléments conducteurs **12, 13** se présentent sous la forme d'une roue métallique montée mobile en rotation sur le support **16.** Ainsi, dans le premier et deuxième mode de réalisation, le bord distal **14, 15** circulaire des éléments conducteurs **12, 13** de contact est de forme arrondie.

Avantageusement, le système **1** de mesure comprend au moins un actionneur (non représenté sur les dessins). L'actionneur peut être un vérin, un bras télescopique ou un bras robotisé par exemple. Le support **16** est agencé sur l'actionneur de sorte que ce dernier est apte à déplacer les éléments conducteurs **12, 13** de contact jusqu'à la bande **2** d'électrode. Cet actionneur est apte à déplacer les éléments conducteurs **12, 13** de contact entre une position de repos dans laquelle ces éléments conducteurs **12, 13** de contact sont en retrait par rapport à la surface de la bande 2 d'électrode et une position de mesure dans laquelle ces éléments conducteurs **12, 13** de contact sont en contact avec la surface de la bande **2** d'électrode.

Dans un mode de réalisation, la distance entre deux bords distaux **14, 15** de contact est réglable de sorte à pouvoir choisir la position et la largeur de la section cible de la surface de la bande **2** d'électrode pour mesurer la conductivité électronique. Dans un mode de réalisation, cette distance est strictement inférieure à la largeur de la couche de matériau actif.

Dans un mode de réalisation illustré par la figure 4, la conductivité électronique de la bande **2** d'électrode est mesurée selon la méthode dite des « 4 pointes » (aussi dite en 4 fils). Cette méthode consiste à mettre en contact quatre bords distaux circulaires (ou pointes) alignés sur la surface de la bande **2** d'électrode. Ensuite, un courant est appliqué entre les deux pointes externes et une tension est mesurée aux bornes des deux pointes internes. La conductivité électronique est calculée à partir de la tension mesurée (par exemple, selon l'équation de Waxman-Smits). Dans un mode de réalisation, les quatre pointes sont placées alignées loin des bords de la couche de matériau actif de la bande **2** d'électrode.

En référence à la figure **4****,** un troisième et un quatrième élément conducteur **17, 18** de contact intégrant, respectivement, un troisième et un quatrième bord distal **23, 24** circulaire peuvent être prévus pour une mesure de la conductivité électronique selon le principe de mesure en quatre pointes. Le troisième bord distal **23** circulaire est destiné à venir en contact sensiblement tangentiel avec la surface de la bande **2** de l'électrode lorsque le premier et le deuxième bord distal **14, 15** circulaire sont en contact avec cette surface. Le quatrième bord distal **24** circulaire est destiné à venir en contact sensiblement tangentiel avec la surface de la bande 2 d'électrode lorsque le premier, le deuxième et le troisième bord **14, 15, 23** distal circulaire sont en contact avec cette surface de sorte à pouvoir mesurer une tension électrique entre le troisième et le quatrième élément conducteur de contact, le troisième et le quatrième bord distal circulaire étant agencés de sorte à venir en contact avec la surface de la bande d'électrode entre le premier et le deuxième bord distal circulaire sensiblement sur une même droite. Le troisième (respectivement, le quatrième) élément conducteur de contact est monté mobile en rotation sur le support **16** de sorte que, lorsque le troisième (respectivement, le quatrième) bord distal circulaire vient en contact sensiblement tangentiel avec la surface de la bande d'électrode défilant entre le premier et le deuxième rouleau rotatif, le troisième (respectivement, le quatrième) élément conducteur de contact soit entrainé en rotation. Le troisième élément **17** conducteur et le quatrième élément **18** conducteur peuvent être de forme annulaire (roue métallique) ou sphérique, comme le premier élément conducteur et le deuxième élément conducteur.

Dans un mode de réalisation, lorsque la bande **2** d'électrode comprend une couche de matériau actif sur ses deux faces, deux systèmes de mesure sont disposés de part et d'autre de la bande **2** d'électrode. Ces deux systèmes de mesure sont, de préférence, disposés en regard l'un de l'autre de part et d'autre de la bande **2** d'électrode pour éviter une éventuelle déformation de cette dernière.

Le dispositif **11** de mesure enregistre les données de conductivité électronique associées à leur position sur la bande d'électrode, dans une base de données et/ou les communique à une unité de contrôle du système **1** de mesure. Dans un mode de réalisation, le dispositif **11** de mesure de conductivité électronique est configuré pour mesurer une conductivité électronique entre deux éléments conducteurs **12, 13** de contact et générer, lorsque la conductivité électronique mesurée est en dehors d'un intervalle prédéfini, un message d'alerte. Ainsi, lorsqu'une anomalie est constatée, une alerte peut être émise à destination de l'opérateur et/ou à une unité de contrôle du système **1** de mesure. Il en résulte, avantageusement, une surveillance automatique continue et en temps réel de la conductivité électronique de la bande **2** d'électrode.

Afin de pouvoir associer la conductivité électronique mesurée à une position précise de la bande **2** d'électrode en défilement, le système **1** de mesure comprend, en outre, un capteur configuré pour mesurer une distance de défilement de la bande **2** d'électrode ou, plus généralement, un moyen apte à situer ou à repérer un défaut de conductivité électronique (par exemple, un marquage physique de type laser ou toute autre technique équivalente). La conductivité électronique mesurée est associée à la distance de défilement mesurée et/ou un horodatage. Il est, ainsi, possible de localiser la position de la bande **2** d'électrode à laquelle une conductivité électronique en dehors d'un intervalle prédéfini a été mesurée.

En se reportant à la figure 5, une méthode de mesure de la conductivité électronique de la bande **2** d'électrode comprend, tel qu'il est décrit ci-dessus, une étape **20** de défilement de la bande **2** d'électrode entre le premier rouleau rotatif et le deuxième rouleau **3, 4** rotatif et une étape **21** de mesure de cette conductivité électronique au moyen du système **1** de mesure présenté ci-dessus disposé entre le premier et le deuxième rouleau **3, 4** rotatif.

Dans un mode de réalisation, cette étape de mesure intervient en amont d'une étape **22** de découpe de la bande **2** d'électrode en une pluralité d'électrodes. Autrement dit, l'étape **22** de découpe de la bande d'électrode en une pluralité d'électrodes est en aval, par rapport au sens de défilement de cette bande d'électrode, de l'étape **21** de mesure.

Avantageusement, les modes de réalisation présentés ci-dessus permettent
- de réduire le surcoût de fabrication des électrodes en détectant en amont tout éventuel défaut et, ainsi, prévenir des défauts de masse des électrodes;
- un contrôle qualité accru en vérifiant en continu la conformité de la conductivité électronique de la bande d'électrode aux objectifs fixés.

## Revendications

1. Ensemble de systèmes de mesure de conductivité électronique d'une bande **(2)** d'électrode pour batterie défilant entre un premier et un deuxième rouleau **(3, 4)** rotatif et comprenant une couche de matériau actif sur ses deux faces, cet ensemble intégrant un premier système **(1)** de mesure de conductivité électronique comprenant
- un dispositif **(11)** de mesure de conductivité électronique ;
- un premier élément conducteur **(12)** de contact connecté audit dispositif **(11)** de mesure, ce premier élément conducteur **(12)** de contact intégrant un premier bord distal **(14)** circulaire destiné à venir en contact sensiblement tangentiel avec une première surface d'une première face desdites deux faces de la bande **(2)** d'électrode ;
- un deuxième élément conducteur **(13)** de contact connecté audit dispositif **(11)** de mesure, ce deuxième élément conducteur **(13)** de contact intégrant un deuxième bord distal **(15)** circulaire destiné à venir en contact sensiblement tangentiel avec la première surface lorsque le premier bord distal **(14)** circulaire est en contact avec ladite première surface de sorte à pouvoir appliquer un courant électrique prédéfini entre le premier élément conducteur **(12)** de contact et le deuxième élément conducteur **(13)** de contact ;
- un support **(16)** pour le premier élément conducteur **(12)** de contact et le deuxième élément conducteur **(13)** de contact,
- le premier élément conducteur **(12)** de contact étant monté mobile en rotation sur ledit support **(16)** de sorte que, lorsque le premier bord distal **(14)** circulaire vient en contact sensiblement tangentiel avec ladite première surface de la bande **(2)** d'électrode défilant entre le premier rouleau **(3)** rotatif et le deuxième rouleau **(4)** rotatif, ledit premier élément conducteur **(12)** de contact soit entrainé en rotation;
- le deuxième élément conducteur **(13)** de contact étant monté mobile en rotation sur ledit support **(16)** de sorte que, lorsque le deuxième bord **(15)** distal circulaire vient en contact sensiblement tangentiel avec ladite première surface de la bande **(2)** d'électrode défilant entre le premier rouleau **(3)** rotatif et le deuxième rouleau **(4)** rotatif, le deuxième élément conducteur **(13)** de contact soit entrainé en rotation ;
ledit ensemble intégrant, en outre, un deuxième système de mesure de conductivité électronique identique audit premier système **(1)** de mesure de conductivité électronique, le premier système **(1)** et le deuxième système étant disposés en regard l'un de l'autre de part et d'autre de la bande **(2)** d'électrode.

2. Ensemble de systèmes selon la revendication précédente,
**caractérisé en ce que** le premier bord distal **(14)** circulaire et/ou le deuxième bord distal **(15)** circulaire du premier système **(1)** sont de forme arrondie.

3. Ensemble de systèmes selon la revendication 1 ou 2, **caractérisé en ce que** le premier élément conducteur **(12)** de contact et/ou le deuxième élément conducteur **(13)** de contact du premier système **(1)** sont de forme sphérique montés en liaison rotule sur ledit support **(16).**

4. Ensemble de systèmes selon l'une quelconque des revendications précédentes, **caractérisé en ce que** la distance entre le premier bord distal **(14)** circulaire et le deuxième bord distal **(15)** circulaire du premier système **(1)** est réglable.

5. Ensemble de systèmes selon l'une quelconque des revendications précédentes, **caractérisé en ce que** le premier système **(1)** comprend, en outre,
- un troisième élément conducteur **(17)** de contact intégrant un troisième bord distal circulaire destiné à venir en contact sensiblement tangentiel avec ladite première surface lorsque le premier et le deuxième bord distal **(14, 15)** circulaire sont en contact avec ladite première surface ;
- un quatrième élément conducteur **(18)** de contact intégrant un quatrième bord distal circulaire destiné à venir en contact sensiblement tangentiel avec la première surface lorsque le premier, le deuxième et le troisième bord distal circulaire sont en contact avec ladite première surface de sorte à pouvoir mesurer une tension électrique entre le troisième élément conducteur **(17)** de contact et le quatrième élément conducteur **(18)** de contact, le troisième et le quatrième bord distal circulaire étant agencés de sorte à venir en contact avec ladite première surface entre le premier et le deuxième bord distal circulaire sensiblement sur une même droite;
- le troisième élément conducteur **(17)** de contact étant monté mobile en rotation sur ledit support **(16)** de sorte que, lorsque le troisième bord distal circulaire vient en contact sensiblement tangentiel avec ladite première surface de la bande **(2)** d'électrode défilant entre le premier rouleau **(3)** rotatif et le deuxième rouleau **(4)** rotatif, le troisième élément conducteur **(17)** de contact soit entrainé en rotation;
- le quatrième élément conducteur **(18)** de contact étant monté mobile en rotation sur ledit support **(16)** de sorte que, lorsque le quatrième bord distal circulaire vient en contact sensiblement tangentiel avec ladite première surface de la bande **(2)** d'électrode défilant entre le premier rouleau **(3)** rotatif et le deuxième rouleau **(4)** rotatif, le quatrième élément conducteur **(18)** de contact soit entrainé en rotation.

6. Ensemble de systèmes selon l'une quelconque des revendications précédentes, **caractérisé en ce que** le dispositif **(11)** de mesure de conductivité électronique du premier système **(1)** est configuré pour
- mesurer une conductivité électronique entre le premier élément conducteur **(12)** de contact et le deuxième élément conducteur **(13)** de contact ;
- générer un message d'alerte lorsque la conductivité électronique mesurée est en dehors d'un intervalle prédéfini.

7. Ensemble de systèmes selon l'une quelconque des revendications précédentes, **caractérisé en ce que** le premier système **(1)** comprend, en outre, un moyen apte à situer un défaut de conductivité électronique.

8. Méthode de mesure de conductivité électronique d'une bande **(2)** d'électrode pour batterie comprenant une couche de matériau actif sur ses deux faces, cette méthode comprenant :
- une étape **(20)** de défilement de ladite bande **(2)** d'électrode entre un premier rouleau **(3)** rotatif et un deuxième rouleau **(4)** rotatif,
- une étape **(21)** de mesure d'une conductivité électronique de la bande **(2)** d'électrode au moyen de l'ensemble de systèmes de mesure de l'une quelconque des revendications précédentes disposé entre le premier rouleau **(3)** rotatif et le deuxième rouleau **(4)** rotatif.

9. Méthode selon la revendication précédente, **caractérisée en ce qu'**elle comprend, en outre, une étape **(22)** de découpe de ladite bande **(2)** d'électrode en une pluralité d'électrodes en aval de l'étape **(21)** de mesure par rapport au sens de défilement de cette bande **(2)** d'électrode.
